(19) **Europäisches Patentamt**

**European Patent Office**

**Office européen des brevets**

(11) **EP 1 534 071 B1**

(12) **EUROPÄISCHE PATENTSCHRIFT**

(45) Veröffentlichungstag und Bekanntmachung des Hinweises auf die Patenterteilung:
**14.12.2005 Patentblatt 2005/50**

(51) Int Cl.⁷: **A01N 37/40**, A01N 31/08, A01N 31/14, A61L 2/18 // (A01N37/40, 31:14, 31:08)

(21) Anmeldenummer: **02779390.0**

(22) Anmeldetag: **20.09.2002**

(86) Internationale Anmeldenummer:
**PCT/EP2002/010583**

(87) Internationale Veröffentlichungsnummer:
**WO 2004/021786 (18.03.2004 Gazette 2004/12)**

(54) **Verwendung eines MITTELs ZUR INAKTIVIERUNG PATHOGENER ERREGER AUF FLÄCHEN, INSTRUMENTEN UND IN KONTAMINIERTEN FLÜSSIGKEITEN**

Use of compositions FOR INACTIVATING PATHOGENIC AGENTS ON SURFACES, INSTRUMENTS AND IN CONTAMINATED FLUIDS

Utilisation des PRODUITS PERMETTANT DE RENDRE INACTIFS DES AGENTS ETIOLOGIQUES PATHOGENES PRESENTS SUR DES SURFACES, DES INSTRUMENTS ET DANS DES LIQUIDES CONTAMINES

(84) Benannte Vertragsstaaten:
**AT BE BG CH CY CZ DE DK EE ES FI FR GB GR IE IT LI LU MC NL PT SE SK TR**
Benannte Erstreckungsstaaten:
**AL LT LV MK RO SI**

(30) Priorität: **05.09.2002 DE 10240985**

(43) Veröffentlichungstag der Anmeldung:
**01.06.2005 Patentblatt 2005/22**

(73) Patentinhaber: **Menno Chemie-Vertrieb GmbH 22850 Norderstedt (DE)**

(72) Erfinder:
• **NEVERMANN, Eugen D-22397 Hamburg (DE)**

• **NEVERMANN, Jan 22848 Norderstedt (DE)**
• **ZERLING, Wolfgang 24568 Kaltenkirchen (DE)**
• **HÖFFLER, Jutta 20249 Hamburg (DE)**

(74) Vertreter: **Becker Kurig Straus Patentanwälte Bavariastrasse 7 80336 München (DE)**

(56) Entgegenhaltungen:
**WO-A-94/17661          DE-B- 1 288 747**

**Beschreibung**

Durch Infektionen, die von Patienten in Krankenhäusern und anderen medizinischen Einrichtungen erworben werden, entstehen große Schäden für die Versichertengemeinschaft und die Volkswirtschaft. Diese als nosokomiale Erkrankungen bezeichneten Infektionen wurden in der Vergangenheit vorwiegend den Bakterien zugeordnet.

Das lag zum einen daran, dass viele Erkrankungen mykologischer oder viraler Genese mangels ausreichender medizinischer Diagnostik nicht erkannt wurden. Zum anderen haben als Folge moderner therapeutischer Maßnahmen aber auch durch Reiseverkehr und globale Verflechtungen Infektionen zugenommen, die durch Viren und Pilze bedingt sind; z.B. die in jüngerer Zeit beobachteten Seuchenzüge in der europäischen Tierhaltung sind ausnahmslos viraler Genese wie die MKS, Aujeszkysche Krankheit sowie die Schweinepest. In Krankenhäusern werden zunehmend virale Erkrankungen z.B. der Norwalk - like Viren, Rotaviren und Adenoviren diagnostiziert, aber auch Pilzinfektionen, die zu Systemmykosen und sekundären Infektionen führen.

Diese neue Situation und der neue Kenntnisstand haben in den letzten Jahren dazu geführt, dass prophylaktische Maßnahmen wie z. B. Desinfektionsverfahren neu überdacht und konzipiert werden müssen. So wird von verschiedenen normgebenden Stellen gefordert, dass neben Bakterien auch besonders widerstandsfähige Pilze (z.B. Aspergillus niger) und Viren (z.B. Poliovirus und Adenovirus) durch die Desinfektion erfasst werden müssen.

Umfassend anwendbare Desinfektionsmittel mit hinreichend viruzider Wirksamkeit gelangen derzeit nur noch stark begrenzt zur Anwendung. Grund dafür sind die Nebenwirkungen der Agenzien. Davon sind insbesondere aldehydische Wirkstoffe betroffen, wie z.B. Formaldehyd, Glutardialdehyd, Succindialdehyd oder Glyoxal und deren aldehydabspaltenden Derivate.

Diese Komponenten galten bisher als klassische Träger einer breiten antimikrobiellen und antiviralen Wirksamkeit in Desinfektionsmittelformullerungen.

Die Agenzien, mit der universellsten Einsatzbreite, - weil auch anwendungstechnisch unproblematisch - im Kampf gegen pathogene Erreger, Formaldehyd und Glutardialdehyd, wurden als giftig eingestuft und stehen im Verdacht kanzerogen zu sein. Bei anderen Aldehyden werden vergleichbare Eigenschaften vermutet.

Dies hat zur Folge, dass die Anwender, wegen des Gefährdungspotenzials, auf aldehydbasierte Desinfektionsmitteln weitgehend verzichten.

Andere zur Verfügung stehende Wirkstoffe sind gegenüber unbehüllten Viren und bestimmten Pilzarten wegen deren besonderen Resistenz nicht bzw. nur begrenzt wirksam oder aufgrund ihrer ungünstigen chemischen und physikalischen Eigenschaften nicht beliebig einsetzbar.

Dies gilt für die Klasse der Per-Verbindungen, für Jod, Chlorabspalter, Alkohole, kat. Tenside, Amphotenside, Phenole, Basen, Säuren und Aktiv-Sauerstoff freisetzende Verbindungen.

Phenolische Verbindungen oder quaternäre Ammoniumverbindungen, deren Wirksamkeit weiterhin durch den Zusatz von 2-Hydroxydiphenylethern gesteigert werden kann, sind beispielsweise aus der DE 1 288 474 bekannt. Ihr Einsatzgebiet liegt in dem Bereich von antimikrobiellen Pudern, Salben, Deodorant-Formulierungen, Handwaschpasten, Reinigungsmittel für Wäschereien, Shampoos etc.

Ein weiteres Anwendungsgebiet von Desinfektionsmitteln, die Phenole in Kombination mit organischen Säuren als Wirkstoff, Ethylenglykoldialkylether und anionische Tenside enthalten, ist die Bekämpfung von Parasiten, insbesondere Würmer und Kokzidien, in der Intensivtierhaltung, wie offenbart in WO 94/17661.

Persäuren z. B. haben ein sehr breit gefächertes antimikrobielles Wirkungsspektrum, sind jedoch wegen ihrer extrem korrodierenden Eigenschaften nur sehr begrenzt einsetzbar. Beträchtliche Probleme ergeben außerdem aus der mangelnden Stabilität dieser Verbindungsklasse.

Auf der Suche nach einer adäquaten Alternative wurde nun überraschend gefunden, dass die Verwendung bestimmter Gemische, umfassend aromatische Hydroxycarbonsäuren und Phenole, die entstandene Lücke zu schließen vermag, dies nicht nur der mikrobiziden Wirksamkeit wegen, sondern auch wegen der günstigen toxikologischen, ökotoxikologischen und materialverträglichen Eigenschaften.

Gegenstand der vorliegenden Erfindung ist die Verwendung von Mitteln gemäß Nauptanspruch zur Inaktivierung von pathogenen Erregern - Bakterien, Pilze und Viren (behüllt und unbehüllt) - auf Flächen und Instrumenten aller Art sowie in kontaminierten Flüssigkeiten. Die Anwendungsgebiete können unterschiedlichster Kategorie sein, z. B. im Bereich von Krankenhäusern, Arztpraxen, Produktionsräumen der Lebensmittelindustrie bis hin zu den Stallungen des Viehzüchters.

Eine synergistische Wirkung zwischen den Komponenten der erfindungsgemäß eingesetzten Desinfektionsmittelgemische wurde nicht nur für die viruziden Eigenschaften nachgewiesen, sondern auch bei den bakteriziden und fungiziden Eigenschaften. Die bakterizide und fungizide Wirkungssteigerung war umso überraschender, weil für die verwendeten Phenole und aromatischen Hydroxycarbonsäuren bereits ausgezeichnete antimikrobielle Wirkungen der Einzelkomponenten bekannt sind.

Bemerkenswert ist außerdem die ungewöhnliche Breite des Wirkungsspektrums, daran zu erkennen, dass hydrophile Picornaviren ebenso sicher inaktiviert werden, wie lipophile Pilze abgetötet werden.

Nachfolgend aufgeführte Beispiele und Tabellen dienen der Erläuterung der vorliegenden Erfindung und dem Nachweis des Synergismus zwischen den Synergisten gemäß Anspruch 1.

Nach F.C. Kull und P.C. Eisman, Applied Microbiology 9, 538-41 (1946) gilt ein Synergismus als nachgewiesen, wenn durch Anwendung der nachstehenden Berechnungsformel das Ergebnis F < 1 zustande kommt.

$$F = QA/Qa + QB/Qb$$

wobei die Zeichen bedeuten:

F < 1    Synergismus
F= 1     Additive Wirksamkeit
F > 1    Antagonismus

Qa =    Menge von A allein zum Endpunkt
Qb =    Menge von B allein zum Endpunkt
QA =    Menge von A in der Mischung mit B
QB =    Menge von B in der Mischung mit A

Beispiele

Beispiel 1

| | |
|---|---|
| Alkylarylsulfonat -Na | 12,0 Gew. Teile |
| Butylmonoglykolsulfonat-Na | 5,0 |
| 4-Chlor-3-methyl-phenol | 15,0 |
| Phosphonobutantricarbonsäure | 1,5 |
| 2-Propylalkohol | 30,0 |
| Wasser entsalzt | 36,5 |

Beispiel 2

| | |
|---|---|
| Alkylarylsulfonat -Na | 12,0 Gew. Teile |
| Butylmonoglykolsulfonat-Na | 5,0 |
| 2-Hydroxybenzoesäure | 6,0 |
| Phosphonobutantricarbonsäure | 1,5 |
| 2-Propylalkohol | 30,0 |
| Wasser entsalzt | 45,5 |

Beispiel 3

| | |
|---|---|
| Alkylarylsulfonat-Na | 12,0 Gew. Teile |
| Butylmonoglykolsulfonat-Na | 5,0 |
| 4-Chlor-3-methylphenol | 15,0 |
| 2-Hydroxybenzoesäure | 6,0 |
| 2-Propylalkohol | 30,0 |
| Phosphonobutantricarbonsäure | 1,5 |
| Wasser entsalzt | 30,5 |

Beispiel 4

| Alkylsulfonat -Na | 10,0 Gew. Teile |
|---|---|
| Cumolsulfonat -Na | 3,0 |
| 2-Phenylphenol | 15,0 |
| β-Resorcinolsäure | 7,0 |
| Ameisensäure | 5, 0 |
| 2-Propylalkohol | 33,0 |
| Wasser entsalzt | 27,0 |

Zum Nachweis des synergistischen Effektes durch die erfindungsgemäßen Kombinationen - die viruziden Eigenschaften betreffend -, wurden die Formulierungsbeispiele 1-3 verwendet.

Das unbehüllte, hydrophile Picornavirus Polio Sabin LSc-2ab diente als Prüfkriterium. Prüfverfahren und Methode wurde gemäß den Regeln der noch vorläufigen europäischen Norm WI 216026 (phase2; step1) durchgeführt.

Versuchsbedingungen:

| Temperatur | 10°C ± 1°C |
|---|---|
| Kontaktzeit | 30 Min. ± 10 Sek. |
| Eiweißbelastung: | 3g/l Rinderserumalbumin und 10g/l Hefeextrakt |

Alle Zahlen in der Tabelle bedeuten, soweit nicht anders bezeichnet, den Infektionstiter (log $ID_{50}$ ml$^{-1}$) nach 30 Min. Einwirkungszeit

( - = nicht mehr nachweisbar)

Tabelle 1

| (Polio Sabin) | | | | | | | |
|---|---|---|---|---|---|---|---|
| | | Konzentrationen [%] | | | | | |
| Muster | Kontrolle | 2% | 3% | 4% | 5% | 6% | 7% |
| Beispiel 1 | 7,7 | 6,9 | 6,3 | 5,8 | 4,3 | 3,9 | 3,0 |
| Beispiel 2 | 7,3 | 6,2 | 5,5 | 4,3 | 3,8 | 2,8 | 2,3 |
| Beispiel 3 | 7,8 | 3,6 | 2,1 | - | - | - | - |

Das Ergebnis ist als ausreichend wirksam zu bewerten, wenn der Infektionstiter um 4 log Stufen reduziert wird, d.h. wenn eine 99,99%ige Reduktion der Infektiosität erreicht ist.

Aus Tabelle 1 folgt, daß die Phenolkomponente in Beispiel 1 bei einer Anwendungskonzentration von 7% wirksam war, während die Hydroxybenzoesäure aus Beispiel 2 eine ausreichende Wirkung bei 6%iger Konzentration zeigte. Das Gemisch aus beiden Komponenten im Formuliezungsbeispiel 3 zeigte bereits bei 2%iger Konzentration eine ausreichende Inaktivierung des Poliovirus.

Setzt man diese Ergebnisse in die Formel von Kull und Eisman ein, so erhält man:

$$F = 2 \times 0{,}06/6 \times 0{,}06 + 2 \times 0{,}15/7 \times 0{,}15 = \underline{0{,}62}$$

Der Zahlenwert 0,62 erbringt somit einen eindeutigen Nachweis für das Vorliegen eines synergistischen Effektes.

(Das Einsetzen der prozentualen Wirkstoffmengen aus den Beispielformulierungen in die Gleichung, führt zu einem konstanten Faktor 1 und ist daher für die Berechnung entbehrlich.)

Ein synergistischer Effekt zur fungiziden Wirksamkeit konnte beispielhaft am besonders resistenten Aspergillus niger dargestellt werden.

Die Untersuchung wurde im quantitativen Suspensionstest nach DIN EN 1650 (phase 2; step1) durchgeführt.

Versuchsbedingungen: DIN EN 1650

Eine Reduktion der Keime um 4 log - Stufen, stellt den erforderlichen Wirksamkeitsnachweis dar.
Die Untersuchungsergebnisse sind in Tabelle 2 abgebildet. Die angegebenen Zahlenwerte sind die Logarithmen ($log_{10}$) der reduzierten Keimzahlen, deren Differenz zur Ausgangskeimzahl den Reduktionsfaktor ergibt.

Tabelle 2

| (Asp. niger) | | | | | |
|---|---|---|---|---|---|
| Beispiele | Konzentrationen [%] | | | | |
| | Prüflösung log(Keimzahl/ml) | 0,25% | 0,5% | 1% | 2% |
| 1 | 7,67 | 4,9 | 3,8 | 3,1 | |
| 2 | 7,67 | 5,2 | 4,2 | 2,8 | |
| 3 | 7,67 | 2,1 | | | |

F = 0,25/1 + 0,25/1 = 0,50

Das vorstehende Rechenergebnis aus den Daten der Tabelle 2 beweist hier ebenfalls einen synergistischen Effekt des Wirkstoffgemisches.
Der Nachweis synergistisch wirksamer Eigenschaften gegen Bakterien wurde mittels einem Gram - positiven und Gram - negativen Testorganismus im quantitativen Suspensionstest nach DIN EN 1276 (phase2; step1) durchgeführt.
Versuchsbedingungen: Einwirkungszeit 20 Min. bei 20°C Eiweißbelastung: 3,0 g/l Rinderserumalbumin
Tabelle 3 zeigt die Ergebnisse mit Escherichia coli.

Tabelle 3

| (E. coli) | | | | | |
|---|---|---|---|---|---|
| Beispiele | Konzentrationen [%] | | | | |
| | Log (Keimzahl/ml) . | 0,25% | 0,5% | 1% | 2% |
| 1 | 8,49 | 4,63 | 4,1 | | |
| 2 | 8,49 | 5,15 | 4,9 | 2,9 | |
| 3 | 8,49 | 3,16 | | | |

F = 0,25/0,5 + 0,25/1 = 0,75

Tabelle 4 zeigt die Ergebnisse mit Staphylococcus aureus.

Versuchsbedingungen: DIN EN 1276

Tabelle 4

| (Staph. aureus) | | | | | |
|---|---|---|---|---|---|
| Beispiele | Konzentrationen [%] | | | | |
| | Log (Keimzahl/ml) | 0,25% | 0,5% | 1% | 2% |
| 1 | 8,4 | 5,9 | 4,8 | 3,3 | |
| 2 | 8,4 | 6,3 | 5,5 | 4,9 | 2,8 |

Tabelle 4   (fortgesetzt)

| (Staph. aureus) | | | | | |
|---|---|---|---|---|---|
| Beispiele | Konzentrationen [%] | | | | |
| 3 | 8,4 | | 5,1 | 3,17 | |

Eine Reduktion der Keime um 4 log Stufen, stellte den erforderlichen Wirksamkeitsnachweis dar.

$$F = 0{,}5/1 + 0{,}5/2 = \underline{0{,}75}$$

In allen Untersuchungen konnten die für den Nachweis des Synergismus erforderlichen mikrobiologischen Ergebnisse erbracht werden, wodurch bewiesen ist, daß die erfindungsgemäßen Formulierungen nach Anspruch 1 gegenüber Bakterien, Pilzsn und Viren synergistisch wirksam sind.

Zum Nachweis einer tuberkuloziden Wirkung im Keimträgerversuch diente die Formulierung nach Beispiel Nr.4 und das Mykobakterium avium Av 56. Die Testbedingungen entsprachen den Vorschriften der Deutschen Veterinärmedizinischen Gesellschaft für den Bereich Tierhaltung (2. Ausgabe 1998).

Keimträger: sterilisierte Lindenholzstücke

(Höhe: 3 mm; Länge: 10 mm; Breite 10 mm)

Tabelle 5

| (Mykobakterium avium) | | | | | |
|---|---|---|---|---|---|
| Beispiel 4 | Einwirkungszeit [Min.] | | | | |
| Konz. [%.] | 30 | 60 | 120 | 180 | 240 |
| 2 | + | + | + | + | + |
| 4 | + | + | - | - | - |
| 5 | + | + | - | - | - |
| 6 | + | + | - | - | - |
| Formalin 3% | + | + | - | - | - |
| Wachstumskontrolle | + | + | + | + | + |

Das in Tab. 5 dargestellte Versuchsergebnis zeigt, daß eine 4%ige Lösung des Formulierungsbeispiels 4 nach 120 Min. Einwirkungszeit die gleiche Wirkung wie eine 3%ige Formalinlösung erzielt. Formalin enthält gemäß DAB 10 35-37% Formaldehyd in Wasser und 10% Methanol, dies entspricht einer wirksamen Konzentration von ca. 1,1 % Aldehyd.

In der Formulierung gemäß Beispiel 4 sind 15% + 7% wirksames Substanzgemisch enthalten, davon gelangen 4% zur Anwendung, was einer tatsächlichen Wirkstoffkonzentration von nur 0,88% entspricht.

Formalin ist bei Keimträgerversuchen mit Lindenholz die allgemein anerkannte Bezugsgröße und Maßstab der Wirksamkeitsprüfung, weil das vergleichsweise kleine Aldehydmolekül besonders gut in die zerklüftete und zerfaserte Struktur des Holzkeimträgers eindringen und wirken kann.

Das Ergebnis zur tuberkuloziden Wirksamkeitsprüfung unterstreicht, ebenso wie die übrigen Ergebnisse, daß die vorliegende Erfindung alle Bedingungen erfüllt, um aldehydbasierte Desinfektionsmittelformulierungen ersetzen zu können.

**Patentansprüche**

1.   Verwendung eines Desinfektionsmittels zur Bekämpfung und Inaktivierung von pathogenen Erregern zur Anwendung auf Flächen und Instrumenten medizinischer und technischer Einrichtungen auf Basis eines synergistisch wirksamen Gemisches von Mono-, Di-, und Trihydroxybenzoesäuren und Phenolen und anionaktiven und nichtionogenen Tensiden als Netzmittel, Hydrotropierungsmitteln, primären und sekundären Alkoholen als Lösungsmittel und aliphatischen Carbonsäuren und Hydroxycarbonsäuren- als pH-Regulatoren sowie Sequestriermitteln, **dadurch gekennzeichnet, dass** es synergistisch wirksame mikrobizide und antivirale Kombinationen aus aromatischen Monohydroxycarbonsäuren, den Dihydroxybenzoesäuren oder den Trihydroxybenzoesäuren, einzeln oder gemischt, und Phenolen in Verbindung mit einem Tensid enthält, das ausgewählt ist aus der Gruppe, bestehend aus Alkylsulfonaten, Alkylarylsulfonsäure, Alkylarylsulfonaten, Alkylarylethersulfaten mit 1-3 EO-Gruppen, Alkylethersulfaten mit 1-3 EO-Gruppen, deren Natrium-, Kalium- und Ammoniumsalze, mit primären oder verzweigten Ketten der Länge C8-C18 als anionisches Tensid und Alkylpolyethylenglykolether mit 3-11 EO- Gruppen als nichtionogenes Tensid, einzeln oder gemischt, wobei das Desinfektionsmittel Butylmonoglykolsulfat, Cumolsulfonat, Toluolsulfonat, Xylolsulfonat, als Natrium-, Kalium- oder Ammoniumsalz einzeln oder als Gemisch als Hydrotropierungsmittel und aliphatische Alkohole und/oder Glykole der Kettenlänge C2-C12 als Lösungsmittel einzeln oder als Gemisch und aliphatische Carbon- und/oder Hydroxycarbonsäuren der Kettenlänge C1 bis C6 einzeln oder als Gemisch als pH-Regulatoren enthält und das Gewichtsverhältnis der Hydrotropierungsmittel und deren Salze, einzeln oder im Gemisch miteinander zwischen 5 und 40 Gew.% bezogen auf das Gesamtgewicht des Desinfektionsmittelkonzentrats liegt.

2.  Verwendung nach Anspruch 1, **dadurch gekennzeichnet, dass** das Gewichtsverhältnis der Hydroxybenzoesäuren (A) zu den Phenolen (B) zwischen 1:9 und 9:1 beträgt und deren Summe zwischen 5 und 40 Gew. % bezogen auf das Gesamtgewicht des Desinfektionsmittelkonzentrates liegt.

3.  Verwendung nach Anspruch 1 oder 2, **dadurch gekennzeichnet, dass** das Gewichtsverhältnis der Alkylsulfonate und/oder Alkylarylsulfate und/oder Ethersulfate und deren Salze (C) mit den Säuren und Phenolen (A+B) im Verhältnis C: (B+A) = 1:9 und 9:1 liegt und deren Summe zwischen 10 und 60% bezogen auf das Gesamtgewicht des Desinfektionsmittelkonzentrates beträgt.

4.  Verwendung nach Anspruch 1, **dadurch gekennzeichnet, dass** das Gewichtsverhältnis der Alkohole einzeln oder im Gemisch miteinander zwischen 5 und 60 Gew.% bezogen auf das Gesamtgewicht des Desinfektionsmittelkonzentrates liegt.

5.  Verwendung nach Anspruch 1, **dadurch gekennzeichnet, dass** das Desinfektionsmittel zwischen 1-8 Gew.% eines oder mehrerer Sequestriermittel vom Typus der Aminoessigsäuren oder Phosphonsäuren und deren jeweilige Derivate enthält.

6.  Verwendung nach einem der Ansprüche 1 bis 5 in wäßrigen, verdünnten Lösungen, die zwischen 0,5 und 10 Gew. % des Desinfektionsmittelkonzentrates enthält.

7.  Verwendung nach einem der Ansprüche 1 bis 6, **dadurch gekennzeichnet, dass** die Phenole ausgewählt sind aus der Gruppe, bestehend aus 2-Isopropyl-5-methylphenol, 2-, 3- oder 4-Methylphenol, Hexylresorcin, 2-Phenylphenol, 2-Methoxyphenol, 3-Methyl-4-chlorphenol, 3,5-Dimethyl-4-chlorphenol, 2-Benzyl-4-chlorphenol einzeln oder gemischt.

8.  Verwendung nach einem der Ansprüche 1 bis 7, **dadurch gekennzeichnet, dass** die aromatische Monohydroxycabonsäure ausgewählt ist aus der Gruppe, bestehend aus 2-; 3-; 4-Hydroxybenzoesäure.

9.  Verwendung nach einem der Ansprüche 1 bis 8, **dadurch gekennzeichnet, dass** die Dihydroxybenzoesäuren ausgewählt sind aus der Gruppe, bestehend aus 2,3-; 2,4-; 2,5-; 2,6; 3,4-; 3,5-Dihydroxybenzoesäure.

10. Verwendung nach einem der Ansprüche 1 bis 9, **dadurch gekennzeichnet, dass** die Trihydroxybenzoesäure ausgewählt ist aus der Gruppe, bestehend aus 2,3,4-Trihydroxybenzoesäure, 2,4,6-Trihydroxybenzoesäure, 3,4,5-Trihydroxybenzoesäure.

## Claims

1.  Use of a disinfectant for the control and inactivation of pathogenic germs for applying to surfaces and instruments of medical and technical establishments, on the basis of a synergistically effective mixture of mono, di, and trihydroxybenzoic acids and phenols and anionic and nonionic surfactants as wetting agents, hydrotropic agents, primary and secondary alcohols as solvents, and aliphatic carboxylic acids and hydroxycarboxylic acids as pH regulators as well as sequestering agents, **characterized in that** it contains synergistically effective microbicidal and antiviral combinations of aromatic monohydroxycarboxylic acids, the dihydroxybenzoic acids, or the trihydroxybenzoic acids individually or mixed, and phenols in combination with a surfactant selected from the group consisting of alkyl sulfonates, alkyl aryl sulfonic acid, alkyl aryl sulfonates, alkyl aryl ether sulfates having 1 to 3 EO groups, alkyl ether sulfates having 1 to 3 EO groups, the sodium, potassium, and ammonium salts thereof with primary or branched chains having a length of $C_8$ to $C_{18}$ as anionic surfactant and alkyl polyethyleneglycol ethers having 3 to 11 EO groups as nonionic surfactant, individually or mixed,

wherein said disinfectant contains butyl monoglycol sulfate, cumenesulfonate, toluenesulfonate, xylenesulfonate as sodium, potassium, or ammonium salt, individually or as a mixture, as hydrotropic agent, and aliphatic alcohols and/or glycols having a chain length of $C_2$ to $C_{12}$ as solvent, individually or as a mixture, and aliphatic carboxylic and/or hydroxycarboxylic acids having a chain length of $C_1$ to $C_6$, individually or as a mixture, as pH regulators, and the weight ratio of the hydrotropic agents and their salts, individually or in a mixture with each other, is between 5 and 40 % by weight, referred to the total weight of the concentrated disinfectant.

2. Use according to claim 1, **characterized in that** the weight ratio of the hydroxybenzoic acids (A) to the phenols (B) is between 1 : 9 and 9 : 1, and their sum is between 5 and 40 % by weight, referred to the total weight of the concentrated disinfectant.

3. Use according to claim 1 or 2, **characterized in that** the weight ratio of the alkyl sulfonates and/or alkyl aryl sulfates and/or ether sulfates and their salts (C) to the acids and phenols (A + B), is in the ratio C : (B + A) = 1 : 9 and 9 : 1, and their sum is between 10 and 60 %, referred to the total weight of the concentrated disinfectant.

4. Use according to claim 1, **characterized in that** the weight ratio of the alcohols, individually or in a mixture with each other, is between 5 and 60 % by weight, referred to the total weight of the concentrated disinfectant.

5. Use according to claim 1, **characterized in that** the disinfectant contains between 1 to 8 % by weight of one or several sequestering agents of the type of aminoacetic acids or phosphonic acids and their respective derivatives.

6. Use according to one of claims 1 to 5 in aqueous, dilute solutions containing between 0.5 and 10 % by weight of the concentrated disinfectant.

7. Use according to one of claims 1 to 6, **characterized in that** the phenols are selected from the group consisting of 2-isopropyl-5-methylphenol, 2-, 3-, or 4-methylphenol, hexylresorcinol, 2-phenylphenol, 2-methoxyphenol, 3-methyl-4-chlorophenol, 3,5-dimethyl-4-chlorophenol, 2-benzyl-4-chlorophenol, individually or mixed.

8. Use according to one of claims 1 to 7, **characterized in that** the aromatic monohydroxycarboxylic acid is selected from the group consisting of 2-, 3-, 4-hydroxybenzoic acid.

9. Use according to one of claims 1 to 8, **characterized in that** the dihydroxybenzoic acids are selected from the group consisting of 2,3-, 2,4-, 2,5-, 2,6-, 3,4-, and 3,5-dihydroxybenzoic acid.

10. Use according to one of claims 1 to 9, **characterized in that** the trihydroxybenzoic acid is selected from the group consisting of 2,3,4-trihydroxybenzoic acid, 2,4,6-trihydroxybenzoic acid, and 3,4,5-trihydroxybenzoic acid.

## Revendications

1. Utilisation d'un produit désinfectant pour éliminer et inactiver des agents pathogènes pour utilisation sur des surfaces et des instruments de dispositifs médicaux et techniques à base d'un mélange efficace d'un point de vue synergique d'acides mono-, di- et trihydroxybenzoïques et de phénols et de tensioactifs anioniques et non ioniques comme agents mouillants, d'agents hydrotropiques, d'alcools primaires et secondaires comme solvants et d'acides carboxyliques aliphatiques et d'acides hydroxycarboxyliques comme régulateurs de pH ainsi que d'agents complexants, **caractérisé en ce qu'**il contient des combinaisons microbicides et antivirales efficaces d'un point de vue synergique d'acides monohydroxycarboxyliques aromatiques, d'acides dihydroxybenzoïques ou d'acides trihydroxybenzoïques, seuls ou en mélanges, et de phénols associés à un tensioactif qui est choisi dans le groupe comprenant les alkylsulfonates, les acides alkylarylsulfoniques, les alkylarylsulfonates, les alkylaryléthersulfates présentant 1 à 3 groupes EO, les sulfates d'alkyléther présentant 1 à 3 groupes EO, leurs sels de sodium, de potassium et d'ammonium, avec des chaînes primaires ou ramifiées de longueur C8 à C18 comme tensioactif anionique et des éthers d'alkylpolyéthylèneglycol présentant 3 à 11 groupes EO comme tensioactifs non ioniques, seuls ou en mélanges, le produit désinfectant contenant du monosulfate de glycol butylique, du sulfonate de curnol, du sulfonate de toluol, du sulfonate de xylol, des sels de sodium, de potassium ou d'ammonium seuls ou en mélange comme agents hydrotropiques et des alcools aliphatiques et/ou glycols présentant une longueur de chaîne de C2 à C12 comme solvants, seuls ou en mélange et des acides carboxyliques aliphatiques et/ou hydroxycarboxyliques présentant une longueur de chaîne de C1 à C6 seuls ou en mélange comme régulateurs de pH et le rapport en poids de l'agent hydrotropique et leurs sels, seuls ou en mélange l'un avec l'autre se situant entre 5 et 40 % en poids par rapport au poids total du concentré d'agent désinfectant.

2. Utilisation selon la revendication 1, **caractérisée en ce que** le rapport en poids des acides hydroxybenzoïques (A) aux phénols (B) se situe entre 1 :9 et 9 :1 et leur somme se situe entre 5 et 40 % en poids par rapport au poids total du concentré d'agent désinfectant.

3. Utilisation selon la revendication 1 ou 2, **caractérisée en ce que** le rapport en poids du sulfonate d'alkyle et/ou du sulfate d'alkyle et/ou du sulfate d'éther et leurs sels (C) avec les acides et phénols (A + B) par rapport à C : (B + A) est de 1 :9 et 9 :1 et leur somme se situe entre 10 et 60 % par rapport au poids total du concentré d'agent désinfectant.

4. Utilisation selon la revendication 1, **caractérisée en ce que** le rapport en poids des alcools, seuls ou en mélange l'un avec l'autre, se situe entre 5 et 60 % en poids par rapport au poids total du concentré d'agent désinfectant.

5. Utilisation selon la revendication 1, **caractérisée en ce que** l'agent désinfectant contient entre 1 à 8 % en poids d'un ou de plusieurs agents complexants du type des acides amino-acétiques ou acides phosphoniques et leurs dérivés.

6. Utilisation selon l'une quelconque des revendications 1 à 5, dans des solutions aqueuses diluées, contenant entre 0,5 et 10 % en poids de concentré d'agent désinfectant.

7. Utilisation selon l'une quelconque des revendications 1 à 6, **caractérisée en ce que** les phénols sont choisis dans le groupe comprenant le 2-isopropyl-5-méthylphénol, le 2-, 3-, ou 4-méthylphénol, l'hexylrésorcine, le 2-phénylphénol, le 2-méthoxyphénol, le 3-méthyl-4-chlorophénol, le 3,5-diméthyl-4-chlorophénol, le 2-benzyl-4-chlorophénol, seuls ou en mélange.

8. Utilisation selon l'une quelconque des revendications 1 à 7, **caractérisée en ce que** l'acide monohydroxycarboxylique aromatique est choisi dans le groupe comprenant les acides 2- ; 3- ; 4-hydroxybenzoïque.

9. Utilisation selon l'une quelconque des revendications 1 à 8, **caractérisée en ce que** les acides dihydroxybenzoïques sont choisis dans le groupe comprenant les acides 2,3- ; 2,4- ; 2,5- ; 2,6- ; 3,4- ; 3,5-dihydroxybenzoïque.

10. Utilisation selon l'une quelconque des revendications 1 à 9, **caractérisée en ce que** l'acide trihydroxybenzoïque est choisi dans le groupe comprenant l'acide 2,3,4-trihydrobenzoïque, 2,4,6-trihydrobenzoïque, 3,4,5-trihydrobenzoïque.